# EUROPEAN PATENT SPECIFICATION

(11) **EP 3 394 639 B1**
(45) Date of publication and mention of the grant of the patent: **19.10.2022**
(21) Application number: 16809023.1
(22) Date of filing: 08.12.2016
(51) Int. Cl.: G01T 1/10, C09K 11/77

(54) **RADIATION DOSIMETER**
STRAHLENDOSIMETER
DOSIMÈTRE DE RAYONNEMENT

(30) Priority: 23.12.2015 EP 15202245
(43) Date of publication of application: 31.10.2018
(73) Proprietor: AGFA NV, 2640 Mortsel (BE)
(72) Inventor: TAHON, Jean-Pierre, 2640 Mortsel (BE); LEBLANS, Paul, 2640 Mortsel (BE); VANDENBROUCKE, Dirk, 2640 Mortsel (BE); STERCKX, Paul, 2640 Mortsel (BE); JUNG, Jürgen, 2640 Mortsel (BE)
(74) Representative: Lingier, Stefaan Frans L.
(86) International application number: PCT/EP2016/080223
(87) International publication number: WO 2017/108427

(56) References cited:
- EP-A2- 1 405 894
- WO-A2-2010/132569
- US-A1- 2007 215 818

## Description

### Technical Field

The invention relates to radiation dosimeters for accurately measuring the amount and spatial distribution of radiation applied from a source of radiation during radiation therapy. The radiation dosimeters are useful in predicting the dose that will be received by a patient during radiation therapies and its location in the patient. These therapies are based on the use of x-rays, gamma-rays, electron beams and particle beams, the latter using hadrons such as protons, carbon ions and the like.

### Background Art

The field of radiation therapy has rapidly evolved over the past years. Whereas originally, patients were exposed to single fields of radiation on the body part to be treated, later evolving to multiple fields from various directions, now very sophisticated radiation generating machines are available, using linear accelerators which rotate around the patient while changing the shape of the beam (using a multi-leaf collimator to match the shape of the beam to the corresponding shape of the tumour), and even the intensity of the beam. This results in potentially very high treatment quality, where dose to the organs to be treated can be maximised, whereas dose to the neighbouring organs which need to be spared ("OAR" , organs at risk) can be minimised.

However, for a radiation therapist or medical physicist in charge of maximising treatment quality and outcomes, it is not intuitively possible to predict nor verify the effective patient dose. Prediction of the dose in all 3 dimensions has to be done, and is implemented in what is called a ' Treatment Planning System' (TPS).

Most of these systems calculate dose on the basis of approximations. With increasing spatial modulation of the dose patterns administered, where some basic assumptions in most of these TPS such as local electronic equilibrium are not met anymore, there can be a discrepancy between predicted dose pattern and the real dose administered to the patient. Also, several other more trivial operator and machine errors are possible, and have happened in the past when treating patients, with dramatic consequences. Therefore, there is a rising demand for executing a pre-check of the dose administration, at least for complex irradiation patterns.

This is done before irradiating the patient, by irradiation of a dosimeter, placed at and around the location where the patient needs to be treated, in a medium that mimicks the patient, often called "phantom", such as a cube or cylinder containing water, or blocks of "solid water", which is a mainly plastic based material. This solid water is easier to handle than real water and yet with a very similar response to radiation. According to this way, it is possible to verify if the TPS result is indeed going to be delivered to the patient, by comparing in each measurement point the dose actually measured with the TPS.

If the deviations are clinically relevant, this would then warrant the adaptation of the treatment plan and/or the machine settings until the desired actual dose pattern is delivered. Currently, a number of technologies are used, each however with significant drawbacks. It is the purpose of the present invention to overcome most of these drawbacks.

A radiation dosimeter has important requirements:
A. Dose accuracy. The absolute accuracy ideally desired is less than 1 per cent absolute error vs. true dose. To further maintain this accuracy over time, it is preferable to have a dosimeter that would need calibration only at long intervals, and/or would be very quick and easy to calibrate. A very important condition to reach accuracy is body equivalence. This is the first and foremost need for a dosimeter: The response should have the same energy dependence as the body tissue. In this context, it is important to note that body equivalence of a certain dosimeter technology is mainly a challenge for low-energy, scattered radiation. At high radiation photon energies (> 0.1 - 1 MeV), interaction of matter with radiation happens mainly through the Compton scattering mechanism, and all atoms from low to high Z have in a first approximation a similar response.

Since the beam energies used in radiation therapy are typically in the megavoltage range (a 6 MeV linac beam enters the body with an average energy in the order of 1.4 MeV; a Cobalt beam will have a similar energy of 1.173 -1.333 MeV, the Z value of the dosimeter material would not matter that much for this radiation.

However, there is a substantial amount of secondary, scattered radiation generated within the body during irradiation, with significantly lower energies, in the (1 - 1000) keV range. At these low radiation photon energies, interaction of matter with radiation happens for a substantial part through the photo-electric effect. This effect however is much more present for high Z materials, and is very roughly proportional to Z³, multiplied by the density of the material.

Since the body consists mainly of water, with a so-called Z_{eff} of 7.22, it is important therefore to have the dosimeter respond also for this low-energy radiation similar to water. The most evident way is to try to approach the value of Z_{eff} of 7.22 in the closest possible way.

To be suitable for being placed inside a phantom as a stack of dosimeter plates in case of 3D radiation dosimetry, and hence behave as human tissue regarding the path of the radiation beam, the attenuation of the radiation beam by the radiation dosimeter should also be very similar to the human tissue.

Moreover, the response to radiation in function of incident beam angle should be also similar to body tissue as a necessary condition for body equivalence. Especially with the current rotating beams, where radiation can hit the patient and thus the dosimeter from all angles, it is extremely important that this condition is met.

Another necessary condition for dose accuracy is *linearity* of the dosimeter response - twice the amount of dose should result in a doubled dose number. Since many dosimeters are inherently non-linear, a calibration might be needed to convert to linear, accurate results.

B. Sufficient spatial resolution. With the ever increasing spatial detail of radiation dose patterns, a spatial resolution of dosimetric measurements should be able to reflect these modulations. At the current state of the art, a resolution in the order of 1 mm is highly desirable, and the trend to higher resolutions is present.

### C. Sufficient dose range (dynamic range)

Traditionally, irradiations are done in several sessions, typically 30 (called "fractions") of a moderate amount of locally applied radiation, e.g. 2 Gy. However, there is a trend to reduce the number of sessions, for clinical and also practical reasons. At this moment, doses per fraction can reach 20 Gy and higher. Therefore, it becomes mandatory to accurately measure doses up to this high dose level. It also becomes clear that at these doses, even small relative errors can be clinically important, more specifically for the OAR, and hence there is more need for QA effort by dosimetry before irradiating.

D. Dose rate independence. The dosimeter should provide an accurate dose reading, regardless of the rate (expressed in Gy/min) at which the dose was delivered.

E. Ease of use. The dosimeter should be easy to use, not requiring any special precautions, special handling etc.

F. Cost effectiveness. To have a low cost of ownership, the dosimeter should be reusable.

Computed Radiography (CR) has been attempted for use in radiation therapy dosimeters. CR systems inherently produce a linear dose-response over several logs of dose, but more importantly, CR systems are reusable and do not require silver halide film and film processors. Conventional CR systems for radiography will in general generate too many photo-electric electrons at low energies (corresponding with scattered radiation during therapy) as compared to body tissue, and are thus not suitable to represent patient dose as they will provide an overestimate, especially in the areas where the primary radiation is weak (penumbra etc.). In 2005, A.J. Olch, Med. Phys., Vol. 32, No. 9, 2005 showed that BaFBrl:Eu²⁺-based computed radiography (CR) storage phosphor films (SPFs) had the potential to be used for two-dimensional megavoltage radiation therapy dosimetry. However, BaFBrl has a high *Z* number (Z_{eff} = 49) which leads to a strong photon energy-dependence and consequently unacceptable measurement accuracy. The over-response to scattered radiation has been effectively reduced by using a thin lead foil on top of the plate (A.J. Olch, Med. Phys., Vol. 32, No. 9, 2005). However, this results in angle-dependence of the response, thus making the system unsuitable for any rotational therapy - which is however common practice at this moment.

Furthermore the response of CR plates will quickly saturate the CR digitizer, resulting in a very limited exposure range. This is specifically the case with installed base digitizers which are designed to handle much lower radiation doses than the doses used in radiation therapy. Hence conventional plates do not allow to cover the required dose range. They lead to saturation in the CR systems at doses, much lower than 30 Gy.

In US8658990, corresponding to the international publication WO2010/132569 A2, a dosimeter is disclosed which includes a storage phosphor based on europium-doped potassium chloride. KCl:Eu²⁺ has potential for use in radiation therapy dosimetry because this material exhibits excellent storage performance and is reusable due to strong radiation hardness. One of the disadvantages of KCl:Eu²⁺ is the high hygroscopicity and the need for encapsulation against ambient moisture. Protective coating technology could be used to overcome this so that after coating, the dosimeter will not be affected by ambient humidity, but means a more complicated production method and an increased cost of production.

Another issue with CR plates is their sensitivity to ambient light, which partly erases the dose signal. This partial erasure makes the dose readout depending of the time between exposure and readout and is a major disadvantage for a radiation dosimeter. US2008/0035859 discloses the use of thick protective layer in CR plates which is designed to be opaque at wavelengths of light that are not used for stimulation and highly transparent at the wavelength used to stimulate the storage phosphor. Because the protective layer is not opaque at the wavelength used to stimulate the storage phosphor, the protective layer must be thick to have an effect on the reduction of image fading. A thick protective layer is moreover a disadvantage if multiple dosimeters have to be stacked in a phantom.

A need exists for a quantitative, reusable, high resolution dosimeter exhibiting almost no energy-dependence in a 6 MV beam, compatible with installed base digitizers, showing a reduced dependency of incident beam angle, a reduced erasure of the registered dose signal due to ambient light without the need of a thick protective layer.

### Summary of invention

It is an object of the present invention to provide a solution for the above stated problems. The object has been achieved by a radiation dosimeter as defined in claim 1.

An additional advantage of the invention according to claim 1, is the absence of the need of using lead foils on top of the dosimeter to reduce the over-response which makes the production of the dosimeter of the invention more straightforward and less costly.

Other features, elements, steps, characteristics and advantages of the present invention will become more apparent from the following detailed description of preferred embodiments of the present invention. Specific embodiments of the invention are also defined in the dependent claims.

### Brief description of drawings

Fig. 1: Stimulated light signal (in arbitrary units) of the radiation dosimeter RD-12, measured by means of an Agfa CR15-X digitizer, in function of the X-ray dose (Gy) received by the dosimeter.
Fig. 2: Stimulated light signal (in arbitrary units) of the radiation dosimeter RD-12, measured by means of an Agfa CR15-X digitizer, in function of the X-ray dose (Gy) received by the dosimeter.

### Description of embodiments

### A. The stimulable phosphor containing layer

### A.1. The stimulable phosphor

The stimulable phosphor according the present invention can be any suitable stimulable phosphor which absorbs radiation and charged particle radiation and temporarily stores the energy of the absorbed radiation. The absorbed energy is measured as luminescence radiation, as visible light and/or ultraviolet radiation, by stimulating the stimulable phosphor by means of stimulating radiation as infra red or visible light. The stimulable phosphors which are employable in the invention are preferably particles, having a particle size d50 between 0.1 and 5µm, more preferably between 0.3 and 5µm, most preferably between 1.5 and 5.0 µm (less than12µm for d99).

Suitable stimulable phosphors are: Bariumfluorohalide phosphors as disclosed in, e.g., US P 4.239.968, DE OS 2 928 245, US-P 4 261 854, US-P 4539 138, US P 4.512.911, EP 0 029 963, US-P 4 336 154, US-P 5 077 144, US-P 4 948 696, Japanese Patent Provisional Publication N•. 55(1980)-12143, Japanese Patent Provisional Publication N•. 56(1981)-116777, Japanese Patent Provisional Publication N•. 57(1982)-23675, US-P 5 089170, US-P 4 532 071, DE OS 3 304 216, EP 0 142 734, EP 0 144 772, US-P 4 587 036, US-P 4 608 190, and EP 0 295 522. BaFBr doped with Eu (BaFBr:Eu) and BaFBrl doped with Eu (BaFBrl:Eu) are preferably suitable.

Ba₁₋ₓSrₓF_{2-a-b}BrₐX_{b} : zA, wherein X is at least one member selected from the group consisting of CI and I; x is in the range 0.10 ≦ 5 x ≦ 0.55 ; a is in the range 0.70 ≦ a ≦ 0.96; b is in the range 0 ≦ b < 0.15 ; z is in the range 10⁻⁷ < z ≦ 0.15, and A is Eu²⁺ or Eu²⁺ together with one or more of the co-dopants selected from the group consisting of Eu³⁺, Y, Tb, Ce, Tm, Dy, Pr, Ho, Nd, Yb, Er, La, Gd and Lu, and wherein fluorine is present stoichiometrically in said phosphor in a larger atom% than bromine taken alone or bromine combined with chlorine and/or iodine, as disclosed in EP 345 903. BaSrFBr is preferably suitable.

Alkali metal phosphors comprising earth alkali metals as disclosed in e.g. US-P 5,028,509 and EP 0 252 991. Alkalimetal phosphors as e.g. CsBr:Eu, RbBr:Tl and KCI:Eu are preferably suitable.

Halosilicate phosphors as disclosed in, e.g. EP 304 121, EP 382 295 and EP 522 619.

Elpasolite phosphors as disclosed in European Application 94201578 filed on June 17, 1994.

### A.2. Method of forming the phosphor containing layer

The stimulable phosphor is to be dispersed as particles into a binder to form a phosphor containing layer. Preferred binders according to the present invention are organic polymers such as polyethylene glycol acrylate, acrylic acid, butenoic acid, propenoic acid, urethane acrylate, hexanediol diacrylate, copolyester tetracrylate, methylated melamine, ethyl acetate, methyl methacrylate, cellulose acetate butyrate, polyalkyl (meth)acrylates, polyvinyl-n-butyral, poly(vinylacetate-co-vinylchloride), poly(acrylonitrile-co-butadiene-co-styrene), poly(vinyl chloride-co-vinyl acetate-co-vinylalcohol), poly(butyl acrylate), poly(ethyl acrylate), poly(methacrylic acid), poly(vinyl butyral), trimellitic acid, butenedioic anhydride, phthalic anhydride, polyisoprene and/or a mixture thereof. Preferably, the binder comprises one or more styrene- hydrogenated diene block copolymers, having a saturated rubber block from polybutadiene or polyisoprene, as rubbery and/or elastomeric polymers. Most preferred binders are copolymers of vinyl isobutyl ether and n-butyl acrylate.

The binder, prior to the dispersing of the stimulable phosphor particles is preferably solubilised into an appropriate solvent. Appropriate solvents are for example lower alcohols such as methanol, ethanol, n-propanol and n-butanol; chlorinated hydrocarbons such as methylene chloride and ethylene chloride; ketones such as acetone, methyl ethyl ketone and methyl isobutyl ketone; esters of lower alcohols with lower aliphatic acids such as methyl acetate, ethyl acetate, propyl acetate and butyl acetate; ethers such as dioxane, ethylene glycol monoethylether and ethylene glycol monoethylether; and mixtures of the above-mentioned compounds.

To the solution of the binder, stimulable phosphor particles can than be added. The addition is preferably performed under stirring and commonly known dispersion techniques can be used to obtain a finely divided coating dispersion.

The weight ratio of binder to stimulable phosphor is equal to or higher than 10, more preferably higher than 15. The coverage of the stimulable phosphor is preferably between 0.10 and 2.50 mg/cm².

The phosphor containing layer may also contain a colorant (see § B.). The colorant can be added during or after the preparation of the coating dispersion, preferably the colorant is added to the solution of the binder, before, at the same time or after adding the stimulable phosphor particles to the solution.

The coating dispersion may contain a dispersing agent to assist the dispersibility of the phosphor particles therein, and also contain a variety of additives such as a plasticizer for increasing the bonding between the binder and the phosphor particles in the phosphor layer. Examples of the dispersing agent include phthalic acid, stearic acid, caproic acid and a hydrophobic surface active agent. Examples of the plasticizer include phosphates such as triphenyl phosphate, tricresyl phosphate and diphenyl phosphate; phthalates; glycolates and polyesters of polyethylene with aliphatic dicarboxylic acids.

The coating dispersion containing the phosphor particles, the binder and the colorant, if present, can be applied evenly to the surface of the substrate to form a layer of the coating dispersion. The coating can be carried out by a conventional method such as a method using a doctor blade, a roll coater or a knife coater. Subsequent to the coating, the formed phosphor containing layer can be dried at ambient temperature or at increased temperature.

Coating is an economically efficient technique of application of one or more layers onto a substrate. By means of coating techniques, the phosphor containing layer can be applied together with intermediate layers and / or top layers (see §D, §E), but also adhesion improving layers etc. Flexible substrates are particularly suitable for a continuous coating process. Moreover, flexible substrates can be available as rolls and they can be wound and un-wound in the production process of coating and drying or curing.

The thickness of the phosphor containing layer is within the range of 10 µm to 1 mm, preferably 20 µm to 500µm, more preferably 25µm to 100µm.

### B. The colorant

The colorant employable in the invention is required to absorb at least a portion of the stimulating light. It is desired that the mean absorbance of the colorant in the region of the stimulation wavelength of the phosphor is as high as possible. It is more preferred that the colorant absorbs radiation originating from ambient light and which can stimulate the stimulable phosphor of the radiation dosimeter of the invention.

Accordingly, the preferred colorant depends at least on the stimulable phosphor employed in the phosphor containing layer of the radiation dosimeter. The colorant must be chosen in such a way that the stimulating radiation of the used stimulable phosphor is efficiently absorbed. Stimulable phosphors such as BaFBr:Eu, BaFCl:Eu, BaFBr_{0.85} I_{0.15}:Eu and BaFI:Eu, requiring stimulated emission in the wavelength region of 300-500 nm when excited with stimulating rays in the wavelength region of 500-700 nm . Employable for such stimulable phosphors is a colorant having a body color ranging from blue to green. Examples of the colorant employed in the invention include the colorants disclosed in U.S. Pat. No. 4,394,581, that is: organic colorants such as Zapon Fast Blue 3G (available from Hoechst AG), Estrol Brill Blue N-3RL (available from Sumitomo Chemical Co., Ltd.), Sumiacryl Blue F-GSL (available from Sumitomo Chemical Co., Ltd.), D & C Blue No. 1 (available from National Aniline), Spirit Blue (available from Hodogaya Chemical Co., Ltd.), Oil Blue No. 603 (available from Orient Co., Ltd.), Kiton Blue A (available from Ciba-Geigy), Aizen Cathilon Blue GLH (available from Hodogaya Chemical Co., Ltd.), Lake Blue A.F.H. (available from Kyowa Sangyo Co., Ltd.), Rodalin Blue 6GX (available from Kyowa Sangyo Co.,Ltd.), Primocyanine 6GX (available from Inahata Sangyo Co., Ltd.), Brillacid Green 6BH (available from Hodogaya Chemical Co., Ltd.), Cyanine blue BNRS (available from Toyo Ink Mfg. Co., Ltd.), Lionol Blue SL (available from Toyo Ink Mfg. Co., Ltd.), and the like; and inorganic colorants such as ultramarine blue, cobalt blue, ceruleanblue, chromium oxide, Ti02-ZnOCoO-NiO pigment, and the like.

Also for stimulable phosphors such als KCl:Eu, requiring stimulation at a radiation between 450 nm and 700 nm, the colorants as described above can be used. Stimulable phosphors such as Al₂O₃, requiring stimulation a radiation between 450 nm and 600 nm will require colorants having a body colour ranging from yellow to red.

The colorant according to the invention can be a dye or pigment.

The colorant can be present in the phosphor containing layer comprising the stimulable phosphor and the binder, and/or in a top layer if present, and/or in an intermediate layer if present. The colorant can also be present in more than one layer if a top layer and/or intermediate layer is present in the dosimeter material. Preferably, the colorant is present in the top layer and more preferably in the top layer and the phosphor containing layer.

The absorption spectrum of the colorant has to cover at least a part of the wavelength range that is suitable for excitation of the stimulable phosphor, considering that depending on all kind of ambient illumination technology employed (such as fluorescent, LED, incadescent, daylight) different spectral power distributions may be present to create the illumination level required for working ergonomics in ambient light. It has been found that the total light absorbance of the layer package consisting of all aforementioned layers, that is applied to the side of the substrate carrying the phosphor containing layer, must be higher than 0.04, preferably higher than 0.06, more preferably higher than 0.10 at stimulation wavelengths of the phosphor, so as to sufficiently protect the stimulated phosphor from losing luminescence signal due to exposure of the dosimeter to ambient light after irradiation with electromagnetic radiation or particle radiation. The level of absorption of the excitation radiation of the phosphor depends on the spectral overlap of dye spectrum and excitation spectrum of the phosphor. Absorbance level of a dye is determined by the absorbance of a block dye, that has the same weighted transmission in view of the excitation spectrum of the phosphor. Block dye is defined to have constant absorbance in the spectral range where the excitation spectrum has a sensitivity of at least 20% of its peak sensitivity value, and no absorption outside.

The colorant provides a total light absorbance of the layers, applied on the side of the substrate which contains the phosphor containing layer, of at least 0.04 at the stimulation wavelengths of the stimulable phosphor. Therefore the amount of colorant is at least 0.008 mg/cm², preferably at least 0.01 mg/cm².

The total absorbance of all the layers can be measured by means of suitable spectro-photometric techniques. The choice of measurement geometry depends on the type of substrate, to which the layers have been applied and follows the guidelines of ASTM E 179. (i) In case of a transparent substrate, total absorbance is either measured indirectly as the complement of the sum of total transmittance and total reflectance, or directly in centre-mount geometry. (ii) If the substrate is highly light reflecting, for example a diffusely reflecting white foil, an additional calculation has to be performed to compensate for the amplifying effect of multiple internal reflections when measuring absorbance through the air interface of a layer that has optical contact to a white diffusely reflecting substrate. Such calculation of internal absorption from externally measured absorption is based on the Saunderson formula as described in ISO 18314-2:2015 (Analytical colorimetry -- Part 2: Saunderson correction, solutions of the Kubelka-Munk equation, tinting strength, hiding power). In thas case absorption is derived from reflection measurements using a spectrophotometer in 45°/0° configuration, in which the specular component is exluded. The Saunderson parameters depend on the refractive index of the layers and the interface roughness and may have to verified for a given layer package by suitable techniques. (iii) If the substrate reveals only a very low level of reflection, for example in the case of a black PET foil, calculation of the total absorbance is difficult due to the absence of reflection. In that case, one will have to split the layer package from the black substrate or to prepare a cross section using a very low angle (ultra low angle microtomy), such that the absorption of the layer package can be measured with a micro spot spectrophotometer in analogy to the characterization of a transparent layer as described before.

### C. The substrate

The substrate for the phosphor containing layer according to the invention, is preferably one which has a low radiation (e.g. X-rays, gamma-rays,...) absorptivity. The low absorptivity for X-rays, gamma rays, charged particle radiation is required in order to achieve a water-like behaviour of the radiation dosimeter, more specifically if used in a stack of dosimeters. It can be either rigid or flexible, such as an aluminium plate, an aluminium foil, a film of polyethylene terephthalate (PET), polyethylene naphthalate (PEN), polyimide (PI), polyethersulphone (PES), a metal foil, a carbon fibre reinforced plastic (CFRP) sheet, glass, flexible glass, triacetate and a combination thereof or laminates thereof. Preferred materials for the substrate to be used in the invention are PET, PEN and glass.

Suitable substrates for the invention also include substrates which are absorbing stimulation radiation of the stimulable phosphor in the phosphor containing layer. In a preferred embodiment of the invention, black coloured substrates can be used to absorb stimulation radiation of the stimulable phosphor because of their high efficiency to absorb light. Black particles, such as fine carbon black powder (ivory black, titanium black, iron black), are suitable.

### D. Intermediate layers

Optionally, intermediate layers can be applied between the substrate and the stimulable phosphor containing layer. These layers can improve the adhesion of the phosphor containing layer to the substrate. But more preferably these intermediate layers can contain a colorant which absorbs the stimulation radiation of the stimulable phosphor in the phosphor containing layer. This can be the same colorant as used in the phosphor containing layer and / or the top layer (see §B). Other functionalities of the intermediate layers can be reflecting or absorbing the emitted light by the stimulable phosphor. Light-reflecting of emitted light can be obtained by an intermediate layer comprising a light-reflecting material such as titanium dioxide. Light-absorbing of emitted light can be obtained by a light-absorbing material such as carbon black or a colorant. Preferably the solid content of carbon black in a light-absorbing intermediate layer is in the range of 3 to 30 (wt.)%. More preferably the range of the solid content of the carbon black is in the range of 6 to 15 (wt.)% and the layer thickness between 5 and15 µm. Further examples of suitable layers can be found in EP1997866A and in WO 2015/091283 A1.

### E. Top layers

Optionally, one or more top-layers can be formed to cover the phosphor containing layer. With top layer is meant, a layer or film which is present at the side of the phosphor containing layer opposite to the substrate. These top-layers can protect the phosphor containing layer against mechanical damage or moisture. Employed as protective layer may be polyester film, polymethacrylate film, nitrocellulose film and cellulose acetate film. Of these, from the viewpoint of transparency as well as strength, stretched films such as polyethylene terephthalate film and polyethylene naphthalate film are preferred, and from the aspect of moisture resistance, metalized films are specifically preferred, which are obtained by applying a thin layer comprised of metal oxides or silicone nitride onto said polyethylene terephthalate film or polyethylene naphthalate film through vacuum evaporation.

The top layer, according to the present invention can also act as a filter layer comprising a colorant which absorbs stimulating radiation of the stimulable phosphor. Colorants which can be used in the top layer, are the same as described above (see §B). Where the top-layer is a film, the top layer can be coloured by dispersing the colorant during the preparation of the film. Alternatively, the top layer can be coloured by dispersing or solubilising the colorant in a binder solution and by coating the solution on top of the phosphor containing layer.

### F. Backing layer

The radiation dosimeter according to the invention may also comprise one or more layers on the substrate at the opposite side of the phosphor containing layer. These layers, denoted as backing layers can be required to further absorb stimulating radiation of the stimulable phosphor and should then also include a colorant, the same as described above. Other functionalities of the backing layer can be to reduce curl of the radiation dosimeter, more specifically if the substrate is a flexible substrate such as a PET film. The backing layer should then include one or more binders, which can be the same as described above in the section of the phosphor containing layer. The backing layer can further contain a coating aid such as a non-ionic surfactant.

### G. Method of therapy

The radiation dosimeters of the present disclosure may be utilized in methods for measuring radiation from a radiation source such as radiation applied during radiation therapy. Radiation therapy comprises photon therapy based on X-rays and gamma-rays, and particle therapy using beams of energetic protons, neutrons, or positive ions for cancer treatment. The most common type of particle therapy is proton therapy. Particle therapy is sometimes referred to, more correctly, as hadron therapy (that is, therapy with particles that are made of quarks).

Radiation therapy requires quality assurance of the dose applied by, for example, directly measuring the dose or accumulated dosages, measuring a dose other than the dose applied to the patient for therapy to verify proper functioning of the radiation therapy system and calibration of the radiation therapy system.

In one embodiment, the method includes applying a dose of radiation in the direction of a dosimeter comprising a phosphor containing layer comprising a stimulable phosphor, preferably a BaFBr:Eu phosphor and a binder with a weight ratio of binder to phosphor of 10 or more and including a blue pigment such as Ultramarine blue, in the phosphor containing layer. The source of X-rays may be a linear accelerator. In various embodiments, the radiation applied may be more than the radiation conventionally applied in radiology. X-ray voltage may be in the kilovoltage or megavoltage ranges. In some embodiments, the x-ray voltage is at least about 0.5 MV or even about 1 MV.

Once the storage phosphor has been irradiated, it may be optically stimulated to emit photons. The phosphor may be stimulated by visible light, preferably by means of a focused laser beam (e.g., red He-Ne laser to stimulate BaFBr:Eu) or focused visible light (e.g. a lamp focused with a monochromator). In one embodiment, the storage phosphor is BaFBr:Eu and is stimulated at a wavelength from about 633 nm. The BaFBr:Eu storage phosphor typically emits an emission spectra with a peak of about 390 nm. Emission spectra may be detected by a spectrofluorometer (e.g., Hitachi F-3010). The intensity of the emission (i.e., the signal) from the storage phosphor may be correlated to a radiation dose. If the correlated radiation dose differs from the dose that was believed to be applied, the dosing system and equipment may be calibrated and corrected to apply the correct dosage. In another embodiment the signal may be used to verify a radiation dose applied to cancerous tissue of a patient. In another embodiment, the signal may be used to quantify the radiation dose received by a radiation worker, functioning as a radiation safety monitor or film badge.

The dose applied and detected by the storage phosphor for calibration or verification may be the same dose applied to the patient to treat a cancerous tissue or may be a dose that was not applied to a patient and was applied only for purposes of calibration and verification.

The storage phosphor as employed in the present invention may be reused many times in contrary to silver halide film or GafChromic film. To erase or reset the storage phosphor after each use, the phosphor may be illuminated with visible light. In one embodiment of the present disclosure, the storage phosphor is reset and a second dose of radiation is applied in the direction of the dosimeter. The storage phosphor may be optically stimulated to emit photons after the second dose of radiation is applied and a second signal is generated based on the amount of photons detected.

### H. Methods for Treating Patients with a Cancerous Tumor

In one aspect of the present disclosure, the radiation dosimeter according to the invention is utilized in a method for treating a patient having a cancerous tumor to predict and verify the effective patient dose. Prediction of the dose in 1, 2 or 3 dimensions is performed in a Treatment Planning System. A targeted dose of radiation is initially defined according to protocols and dosages known and determinable within the radiation oncology field. The targeted dose of radiation is first verified by applying a dose of radiation in the direction of the dosimeter including the phosphor containing layer comprising the stimulable phosphor and binder in a weight ratio of binder to phosphor of 10 or more and further comprising a colorant absorbing stimulation radiation of said phosphor. By measuring the stimulated emission of the phosphor in the dosimeter, it is possible to verify if the Treatment Planning System result is indeed going to be delivered to the patient, by comparing in each measurement point the dose actually measured with the Treatment Planning System prediction.

The dose applied and detected by the storage phosphor may be the same dose applied to the patient to treat a cancerous tissue or may be a dose that was not applied to a patient and was applied only for purposes of calibration and verification.

The radiation treatment utilized for treatment may be, for example, external beam radiotherapy (2DXRT), external beam radiotherapy (EBRT), 3D conformal radiotherapy (3DCRT), Volume Modulated Arc Therapy (VMAT) or Intensity-Modulated Radiation Therapy (IMRT). The dosimeter may be utilized in three-dimensional applications by stacking multiple radiation dosimeters. The targeted dose of radiation applied in a treatment session may be at least about 0.5 Gy and, in other embodiments, is at least about 1.5 Gy, from about 1.5 to about 3 Gy or from about 1.8 to about 2 Gy. In some embodiments, the total dose of radiation applied to the patient is fractionated meaning a partial dose of radiation is applied many times (e.g., from about 1.5 to about 3 Gy) until the total dose is achieved. The total dose of radiation may be from about 5 Gy to about 80 Gy.

While the present invention will hereinafter in the examples be described in connection with preferred embodiments thereof, it will be understood that it is not intended to limit the invention to those embodiments.

### I. Examples

### I.1. Materials

- Baysilon: Baysilone Paint additive MA from Bayer
- CAB381-2: 20(wt.)% solution of Cellulose Acetate Butyrate (CAB-381-2) from Eastman in MEK. Prepared by stirring for 8 hours at 1600 rpm and filtering with Filter AU09E11 NG after stirring.
- BaFBr:Eu: stimulable phosphor particles of Ba_{0.921}Sr_{0.077}Br_{0.8}F_{1.03}I_{0.17}:0.002Eu having a particle size d50 of between 1.5 and 5.0 µm produced as described in US 2007/0075270 A1 [0065 - 0085].
- Acronal 500L: acrylic polymer from BASF
- PET-foil: White PET substrate: polyethylene terephthalate (PET) film with a thickness of 0.19 mm, obtained from Mitsubishi, trade name Hostaphan WO
- UMB: Ultramarine blue CM121 from HOLLIDAYS DYES AND CHEMICALS LTD

### I.2. Measurements

### I.2.1. Resistance to ambient light

The samples of dosimeter material were exposed to a X-ray source: Siefert at a distance of 2,25m, without a filter. The current settings were: 10 mA at 77kV during 180s. After the irradiation with X-rays, the samples were partially (50%) covered with a black PE-foil and then exposed during 10 minutes to ambient light originating from a luminescence tube at a level of 3.4 Lux, measured with a LMT Pocket-Lux 2 SN 4198. The luminescence of the part which was exposed to ambient light and the part which was covered by the black PE-foil was measured in a research CR reader under standardized conditions. The time between the irradiation and start of the reading out of the dosimeter is fixed at 10 minutes. The decrease in luminescence was expressed as a ratio of the luminescence measured in the exposed part to the luminescence measured in the unexposed part of the sample to ambient light.

### I.2.2. Light absorbance measurements of the layers onto the substrate

The dosimeters of the example were prepared using white diffusely reflecting PET as substrate. In order determine the total light absorbance of the layers, applied onto the substrate, reflection measurements were performed by means of a Gretag Densitometer SPM50 in a 45°/0° configuration. From these measurements of absorbance of the layers applied onto the white the substrate, the total light absorbance of the layers without the contribution of the substrate, was calculated making use of the Inverse Saunderson formula.

### 1.2.3 Measurement of the photostimulated emission

Exposure to X-rays was performed on Varian Linac 2100C/D Varian Medical Systems, Palo Alto, CA) using clinical photon beams of 6 and 10MV. The dosimeter was positioned in a Multicube phantom or was sandwiched between a stack of solid water (acrylic) RW3 slabs. To obtain a uniform irradiation field a stack of RW3 slabs (30 × 30 × 20 cm³) was positioned on the floor. The source to surface distance (SSD) was 213 cm and a 40 ×40 cm² field delivered the radiation dose. Other irradiations were performed using an isocentric setup (SSD_{Multicube} = 89 cm, SSD_{RW3} = 90 cm).

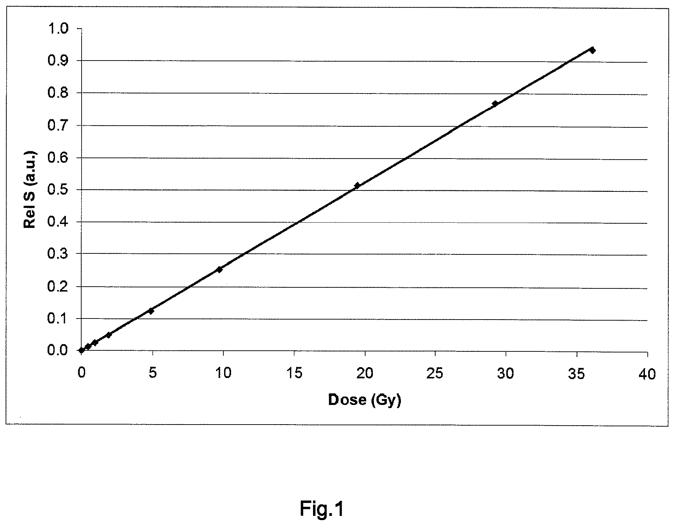

Measurement of photostimulated emission (S) of the stimulable phosphor was performed by an Agfa CR15-X digitizer in SR mode with 200 µm pixel size. The reader was coupled to a standard NX workstation. The time between exposure to X-rays and the measurement of the level of S was 4 min. Unprocessed images were exported from the NX workstation as "Native" ("For processing") and analyzed with ImageJ software. As the digitizer uses a SQRT amplifier, images are squared and in a standard region of interest (ROI) the average pixel value is measured. Plotted against the dose (Fig. 1), a linear relation is obtained up to > 40 Gy.

### 1.3. Example 1

### 1.3.1. Preparation of radiation dosimeters RD-01 - RD-10

The coating dispersion of the phosphor containing layer was prepared as follows: 0.08 g of Baysilon (10% pre-disolved in MEK), 17.92 g of Propylacetate and 45 g of CAB381-2 (20% predisolved in MEK) were mixed by stirring at 1800 rpm with a Disperlux stirrer for 1 minute. The BaFBr phosphor and UMB were then added under stirring and thereafter the dispersion was stirred for another 5 minutes a rate of 1800 r.p.m. After this step Acronal 500L (25% in mixture MEK / Methoxy Propanol / Ethylacetate) was added. The dispersion was then stirred for another 5 minutes at a rate of 1800 rpm.

The dispersion was coated with an Elcometer adjustable Baker film applicator type 3530 at a coating rate of 1.4 cm/s per minute onto PET-foil as substrate with an automatic film applicator 4340/SP from Braive Instruments. The coating dispersions were coated with a coating knife at a wet layer thickness of 200µm and 500µm and dried at room temperature during 5 minutes. The coverage of stimulable phosphor, binders and colorant (= UMB) are listed in Table 1.

**Table 1**

| mg/cm² | UMB (mg/cm²) | BaFBr (mg/cm²) | CAB 381-2 (mg/cm²) | Acronal 500L (mg/cm²) |
|---|---|---|---|---|
| RD-01 | 0.000 | 0.132 | 1.184 | 1.184 |
| RD-02 | 0.000 | 0.395 | 3.553 | 3.553 |
| RD-03 | 0.009 | 0.131 | 1.181 | 1.179 |
| RD-04 | 0.026 | 0.393 | 3.543 | 3.538 |
| RD-05 | 0.021 | 0.130 | 1.177 | 1.172 |
| RD-06 | 0.063 | 0.391 | 3.530 | 3.516 |
| RD-07 | 0.035 | 0.129 | 1.171 | 1.164 |
| RD-08 | 0.106 | 0.388 | 3.514 | 3.492 |
| RD-09 | 0.057 | 0.128 | 1.164 | 1.151 |
| RD-10 | 0.171 | 0.384 | 3.491 | 3.454 |

### 1.3.2. Resistance to ambient light

Radiation dosimeters RD-01 to RD10 were subjected to measurements of the resistance to ambient light measurements as described in § I.2.1. The results are summarised in Table 2 together with the light absorbance, measured as described in § I.2.2.

**Table 2**

| | Comparison / Invention | Absorbance at 520 nm | % of luminescence after 5' exposure to ambient light |
|---|---|---|---|
| RD-01 | COMP | 0.00 | 69 |
| RD-02 | COMP | 0.00 | 72 |
| RD-03 | INV | 0.04 | 81 |
| RD-04 | INV | 0.11 | 87 |
| RD-05 | INV | 0.06 | 84 |
| RD-06 | INV | 0.15 | 89 |
| RD-07 | INV | 0.09 | 88 |
| RD-08 | INV | 0.22 | 93 |
| RD-09 | INV | 0.14 | 91 |
| RD-10 | INV | 0.32 | 98 |

From Table 2 it is clear that radiation dosimeters having a colorant which absorbs light at the wavelengths of stimulation radiation of the BaFBr, show an increased resistance to exposure to ambient light.

### Example 2

### Preparation of radiation dosimeters RD-11 to RD-13

The coating dispersions were prepared the same way as described in Example 1 with the exception of the amount of BaFBr and UMB. The composition of the radiation dosimeters are summarised in Table 3 . Prior to coating of the PET-foil, a backing layer was coated on the PET-foil opposite to the side on which the phosphor containing layer was to be applied. 100 g of the coating solution of the backing layer consists of 45 g Cab 381-2, 0.08 g of Baysilon, 36 g of Acronal 500L and 18.92 g of propylacetate. The coating solution of the backing layer was applied using a coating knife at a layer thickness of 200 µm.

**Table 3**

| mg/cm² | UMB (mg/cm²) | BaFBr (mg/cm²) | CAB 381-2 (mg/cm²) | Acronal 500L (mg/cm²) |
|---|---|---|---|---|
| RD-11 | 0.009 | 0.259 | 1.167 | 1.165 |
| RD-12 | 0.011 | 0.327 | 1.471 | 1.469 |
| RD-13 | 0.018 | 0.136 | 1.225 | 1.221 |

Radiation dosimeters RD-11 to RD-13 were subjected to measurements of resistance to ambient light as described in § I.2.1. The results are summarised in Table 3 together with the light absorbance, measured as in § I.2.2.

**Table 4**

| | Comparison / invention | Absorbance at 520 nm | % of luminescence after 5' exposure to ambient light |
|---|---|---|---|
| RD-11 | INV | 0.11 | 81.7 |
| RD-12 | INV | 0.11 | 82.2 |
| RD-13 | INV | 0.09 | 81.3 |

From Table 4 and Table 2 it is clear that radiation dosimeters having a colorant which absorbs light at the wavelengths of stimulation radiation of the BaFBr, show an increased resistance to exposure to ambient light.

The radiation dosimeter RD-11 was subjected to X-ray doses and the luminescence signal was measured as described in § I.2.3. The result of the measurements is illustrated in fig. 1, which is a graphical representation of the response of the dosimeter after an X-ray dose. As can be seen, a linear response is observed up to 40 Gy.

### Example 3

### Preparation of radiation dosimeters RD-14 to RD-17

The phosphor containing layer is prepared and coated, the same way as in Example 1, but with different concentrations of UMB and BaFBr. After drying a top layer was coated on top of the phosphor containing layer. The coating dispersion of the top-layer was prepared and coated, the same way as described in Example 1, but without BaFBr. The composition of the phosphor containing layer and the top-layer is summarised in Table 5.

**Table 5**

| | UMB in phosphor cont. layer (mg/cm²) | UMB in top-layer (mg/cm²) | BaFBr in phosphor cont. layer (mg/cm²) | CAB 381-2 in phosphor cont.layer & top-layer (mg/cm²) | Acronal 500L in phosphor cont.layer & top-layer (mg/cm²) |
|---|---|---|---|---|---|
| RD-14 | 0 | 0 | 0.146 | 1.352 | 1.352 |
| RD-15 | 0 | 0.02 | 0.148 | 1.368 | 1.368 |
| RD-16 | 0.01 | 0.01 | 0.156 | 1.444 | 1.444 |
| RD-17 | 0.02 | 0 | 0.146 | 1.352 | 1.352 |

Radiation dosimeters RD-14 to RD-17 were subjected to measurements of resistance to ambient light measurements as described in § I.1.2. The results are summarised in Table 6 together with the absorbance measured as in § I.2.2.

**Table 6**

| | comparison /invention | Absorbance at 520 nm | % of luminescence after 5' exposure to ambient light |
|---|---|---|---|
| RD-14 | COMP | 0.00 | 54.0 |
| RD-15 | INV | 0.05 | 78.1 |
| RD-16 | INV | 0.05 | 74.4 |
| RD-17 | INV | 0.05 | 74.1 |

As can be seen in Table 6, the use of a colorant in only a top-layer or in both the top-layer and the phosphor-containing layer, or in only a phosphor containing layer, results in a significant increase in resistance to ambient light with respect to the radiation dosimeter without colorant.

### Example 4

For proton exposure tests the dosimeter RD-06 was sandwiched between a stack of CIRS solid water plates (2 plates of 30 × 30 × 20 cm³ / RW3) with the dosimeter edge matched to the proximal surface of the plastic plates. The stack was positioned in-line with the proton beam axis, but with a tilt of 3° in order to minimize the impact of the difference in proton stopping power between the interfacing media.

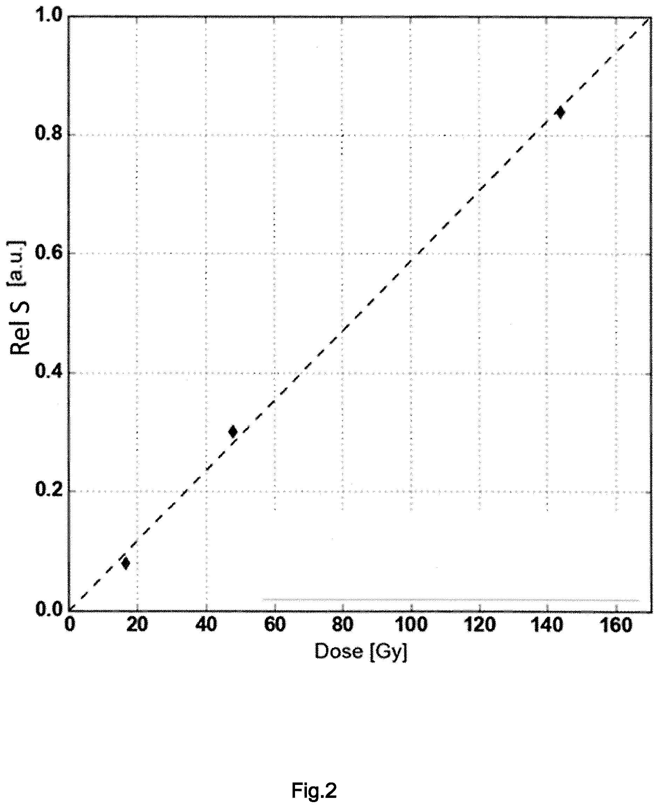

Proton exposures with fluences of 2.56, 7.5 and 22.5 x109 protons/cm² were performed at Université Catholique de Louvain-la-Neuve in Belgium, in a 62 MeV non-modulated proton beam produced by a superconducting cyclotron "CYCLONE110". In contrast to Gafchromic film, the dosimeter RD-06 had a very high dynamic (dose) range allowing measurement of the distal fall-off as well as the entrance and Bragg-peak profile. Moreover, the dynamic range of the CR system as estimated by linear extrapolation is ca. 170 Gy as can be seen in Fig. 2.

## Claims

1. A radiation dosimeter for measuring the dose of radiation applied during radiation therapy, comprising:
a substrate; and
a phosphor containing layer comprising a stimulable phosphor and a binder on a side of the substrate; and
a colorant; and
optionally a layer in contact with the phosphor containing layer;
**characterised in that** the weight ratio of binder to phosphor in the phosphor containing layer is 10 or higher and that the colorant provides a total light absorbance of the layers, applied on the side of the substrate comprising the phosphor containing layer, of at least 0.04 at the stimulation wavelengths of the stimulable phosphor.

2. The radiation dosimeter according to claim 1, wherein the stimulable phosphor is BaFBr(I):Eu, BaSrFBr:Eu or CsBr:Eu.

3. The radiation dosimeter according to any of the preceding claims, wherein the colorant is present in the phosphor containing layer.

4. The radiation dosimeter according to any of the preceding claims, wherein the optional layer is a top layer present on top of the phosphor containing layer.

5. The radiation dosimeter according to claim 4, wherein the top layer contains the colorant.

6. The radiation dosimeter according to any of the preceding claims 1 to 3, wherein the optional layer is an intermediate layer present between the phosphor containing layer and the substrate.

7. The radiation dosimeter according to claim 6, wherein the intermediate layer contains the colorant.

8. The radiation dosimeter according to claim 5, wherein the colorant is present in the phosphor containing layer and in the top layer

9. The radiation dosimeter according to any of the preceding claims wherein the weight ratio of binder to phosphor in the phosphor containing layer is 15 or higher.

10. The radiation dosimeter according to any of the preceding claims wherein the amount of the colorant is at least 0.008 mg/cm².

11. The radiation dosimeter according to any of the preceding claims wherein the substrate consists of a white PET foil.

12. The radiation dosimeter according to any of the preceding claims wherein the substrate consists of a black coloured substrate.

13. The radiation dosimeter according to any of the preceding claims, wherein the colorant is a pigment.

14. A method of measuring a radiation dose, comprising the steps of:
(a) exposing the dosimeter as defined in any of the claims 1 to 8, to radiation; and
(b) stimulating the irradiated dosimeter with visible light such as to emit light; and
(c) measuring the intensity of the emitted light.

15. The method according to claim 14 wherein the radiation consists of protons.

## Patentansprüche

1. Ein Strahlendosimeter zur Messung der während einer Strahlungstherapie angewandten Strahlendosis, umfassend:
ein Substrat, und
eine an einer Seite des Substrats aufgetragene, Leuchtstoff enthaltende Schicht, die einen stimulierbaren Leuchtstoff und ein Bindemittel enthält, und
ein Farbmittel, und
gegebenenfalls eine Schicht in Berührung mit der Leuchtstoff enthaltenden Schicht,
**dadurch gekennzeichnet, dass** das Gewichtsverhältnis von Bindemittel zu Leuchtstoff in der Leuchtstoff enthaltenden Schicht bei mindestens 10 liegt und das Farbmittel den Schichten, die auf die die Leuchtstoff enthaltende Schicht umfassende Seite des Substrats aufgetragen sind, ein Gesamtlichtabsorptionsvermögen von mindestens 0,04 bei den Stimulationswellenlängen des stimulierbaren Leuchtstoffes verleiht.

2. Der Strahlendosimeter nach Anspruch 1, wobei der stimulierbare Leuchtstoff BaFBr(I):Eu, BaSrFBr:Eu oder CsBr:Eu ist.

3. Der Strahlendosimeter nach einem der vorstehenden Ansprüche, wobei das Farbmittel in der Leuchtstoff enthaltenden Schicht enthalten ist.

4. Der Strahlendosimeter nach einem der vorstehenden Ansprüche, wobei die eventuelle Schicht eine auf der Leuchtstoff enthaltenden Schicht vorliegende Deckschicht ist.

5. Der Strahlendosimeter nach Anspruch 4, wobei die Deckschicht das Farbmittel enthält.

6. Der Strahlendosimeter nach einem der vorstehenden Ansprüche 1 bis 3, wobei die eventuelle Schicht eine zwischen der Leuchtstoff enthaltenden Schicht und dem Substrat vorliegende Zwischenschicht ist.

7. Der Strahlendosimeter nach Anspruch 6, wobei die Zwischenschicht das Farbmittel enthält.

8. Der Strahlendosimeter nach Anspruch 5, wobei das Farbmittel in der Leuchtstoff enthaltenden Schicht und in der Deckschicht enthalten ist.

9. Der Strahlendosimeter nach einem der vorstehenden Ansprüche, wobei das Gewichtsverhältnis von Bindemittel zu Leuchtstoff in der Leuchtstoff enthaltenden Schicht bei mindestens 15 liegt.

10. Der Strahlendosimeter nach einem der vorstehenden Ansprüche, wobei die Menge Farbmittel bei mindestens 0,008 mg/cm² liegt.

11. Der Strahlendosimeter nach einem der vorstehenden Ansprüche, wobei das Substrat aus einer weißen PET-Folie besteht.

12. Der Strahlendosimeter nach einem der vorstehenden Ansprüche, wobei das Substrat aus einem schwarzgefärbten Substrat besteht.

13. Der Strahlendosimeter nach einem der vorstehenden Ansprüche, wobei das Farbmittel ein Pigment ist.

14. Ein Verfahren zur Messung einer Strahlendosis, das die folgenden Schritte umfasst:
(a) Bestrahlung des nach einem der Ansprüche 1 bis 8 definierten Dosimeters, und
(b) Stimulieren des bestrahlten Dosimeters mit sichtbarem Licht, um Lichtemission auszulösen, und
(c) Messen der Intensität des emittierten Lichtes.

15. Das Verfahren nach Anspruch 14, wobei die Strahlung aus Protonen besteht.

## Revendications

1. Dosimètre de rayonnement destiné à la mesure de la dose de rayonnement appliquée lors d'une radiothérapie, comprenant:
un substrat, et
une couche contenant un luminophore appliquée sur un côté du substrat et contenant un luminophore stimulable et un liant, et
une matière colorante, et
éventuellement une couche en contact avec la couche contenant un luminophore,
**caractérisé en ce que** le rapport pondéral de liant à luminophore dans la couche contenant un luminophore s'élève à au moins 10 et que la matière colorante confère une absorbance de lumière totale d'au moins 0,04 aux longueurs d'onde de stimulation du luminophore stimulable aux couches appliquées sur le côté du substrat contenant la couche contenant un luminophore.

2. Dosimètre de rayonnement selon la revendication 1, **caractérisé en ce que** le luminophore stimulable est le BaFBr(I):Eu, le BaSrFBr:Eu ou le CsBr:Eu.

3. Dosimètre de rayonnement selon l'une quelconque des revendications précédentes, **caractérisé en ce que** la matière colorante est contenue dans la couche contenant un luminophore.

4. Dosimètre de rayonnement selon l'une quelconque des revendications précédentes, **caractérisé en ce que** la couche éventuelle est une couche supérieure appliquée sur la couche contenant un luminophore.

5. Dosimètre de rayonnement selon la revendication 4, **caractérisé en ce que** la couche supérieure contient la matière colorante.

6. Dosimètre de rayonnement selon l'une quelconque des revendications précédentes 1 à 3, **caractérisé en ce que** la couche éventuelle est une couche intermédiaire qui se situe entre la couche contenant un luminophore et le substrat.

7. Dosimètre de rayonnement selon la revendication 6, **caractérisé en ce que** la couche intermédiaire contient la matière colorante.

8. Dosimètre de rayonnement selon la revendication 5, **caractérisé en ce que** la matière colorante est contenue dans la couche contenant un luminophore et dans la couche supérieure.

9. Dosimètre de rayonnement selon l'une quelconque des revendications précédentes, **caractérisé en ce que** le rapport pondéral de liant à luminophore dans la couche contenant un luminophore s'élève à au moins 15.

10. Dosimètre de rayonnement selon l'une quelconque des revendications précédentes, **caractérisé en ce que** la quantité de matière colorante s'élève à au moins 0,008 mg/cm².

11. Dosimètre de rayonnement selon l'une quelconque des revendications précédentes, **caractérisé en ce que** le substrat est composé d'une feuille de PET blanche.

12. Dosimètre de rayonnement selon l'une quelconque des revendications précédentes, **caractérisé en ce que** le substrat est composé d'un substrat de couleur noire.

13. Dosimètre de rayonnement selon l'une quelconque des revendications précédentes, **caractérisé en ce que** la matière colorante est un pigment.

14. Procédé destiné à la mesure d'une dose de rayonnement, comprenant les étapes consistant à:
(a) exposer à du rayonnement le dosimètre tel que défini selon l'une quelconque des revendications 1 à 8, et
(b) stimuler le dosimètre irradié avec de la lumière visible afin de provoquer l'émission de lumière, et
(c) mesurer l'intensité de la lumière émise.

15. Procédé selon la revendication 14, **caractérisé en ce que** le rayonnement est composé de protons.
